(19) Europäisches Patentamt
European Patent Office
Office européen des brevets

(11) **EP 4 229 040 B1**

(12) **EUROPEAN PATENT SPECIFICATION**

(45) Date of publication and mention of the grant of the patent:
**25.02.2026 Bulletin 2026/09**

(21) Application number: **21790200.6**

(22) Date of filing: **11.10.2021**

(51) International Patent Classification (IPC):
***C07D 221/04*** *(2006.01)*

(52) Cooperative Patent Classification (CPC):
**C07D 221/04**

(86) International application number:
**PCT/EP2021/078025**

(87) International publication number:
**WO 2022/078945 (21.04.2022 Gazette 2022/16)**

(54) **ASYMMETRIC TRANSFER HYDROGENATION OF 2-ARYL SUBSTITUTED BICYCLIC PYRIDINE KETONES IN PRESENCE OF A CHIRAL RUTHENIUM CATALYST**

ASYMMETRISCHE TRANSFERHYDRIERUNG VON 2-ARYL-SUBSTITUIERTEN BICYCLISCHEN PYRIDINKETONEN IN GEGENWART EINES CHIRALEN RUTHENIUM-KATALYSATORS

HYDROGÉNATION PAR TRANSFERT ASYMÉTRIQUE DE PYRIDINE CÉTONES BICYCLIQUES À SUBSTITUTION 2-ARYLE EN PRÉSENCE D’UN CATALYSEUR CHIRAL AU RUTHÉNIUM

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.10.2020 EP 20201628**

(43) Date of publication of application:
**23.08.2023 Bulletin 2023/34**

(73) Proprietor: **Bayer Aktiengesellschaft**
**51373 Leverkusen (DE)**

(72) Inventors:
- **SCHOTES, Christoph**
  **40223 Düsseldorf (DE)**
- **GOUDEDRANCE, Sébastien**
  **68510 Sierentz (FR)**
- **SPINDLER, Felix**
  **4656 Starrkirch-Wil (CH)**
- **FUCHS, Beat**
  **6010 Kriens (CH)**

(74) Representative: **BIP Patents**
**c/o Bayer Intellectual Property GmbH**
**Alfred-Nobel-Straße 50**
**40789 Monheim am Rhein (DE)**

(56) References cited:
- **ANU NAIK ET AL: "Iron(II)-bis(isonitrile) complexes: novel catalysts in asymmetric transfer hydrogenations of aromatic and heteroaromatic ketones", CHEMICAL COMMUNICATIONS, vol. 46, no. 25, 2010, pages 4475 - 4477, XP055021267, ISSN: 1359-7345, DOI: 10.1039/c0cc00508h**
- **XIE YINJUN ET AL: "Design and synthesis of new chiral pyridine-phosphite ligands for the copper-catalyzed enantioselective conjugate addition of diethylzinc to acyclic enones", TETRAHEDRON ASYMMETRY, vol. 20, no. 12, 2009, pages 1425 - 1432, XP026281806, ISSN: 0957-4166, [retrieved on 20090626], DOI: 10.1016/J.TETASY.2009.05.014**
- **VIMAL PAREKH ET AL: "Ether-tethered Ru(II)/TsDPEN complexes; synthesis and applications to asymmetric transfer hydrogenation", CATALYSIS SCIENCE & TECHNOLOGY, vol. 2, no. 2, 2012, pages 406 - 414, XP055240917, ISSN: 2044-4753, DOI: 10.1039/C1CY00364J**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 229 040 B1

## Description

The invention relates to a process for preparing optically active 2-aryl substituted 6,7-dihydro-5H-cyclopenta[b] pyridin-7-ols comprising asymmetric transfer hydrogenation of the corresponding ketones in presence of a ruthenium catalyst comprising a chiral diamine or amino alcohol ligand.

It is known from WO 2019/185541 that chiral aryl-substituted bicyclic pyridine-phosphinites are excellent (P,N)-ligands for the iridium-catalyzed enantioselective hydrogenation of 4-substituted *N*-acetyl-dihydroquinolines. By means of these ligands, the resulting 4-substituted N-acetyl-tetrahydroquinolines can be obtained in high yields and excellent enantioselectivity (up to 98% *ee*). Subsequent rearrangement gives access to the corresponding 4-aminoindane derivatives (EP 0 654 464), which are important intermediates for the preparation of various N-indanyl heteroaryl carboxamides having fungicidal activity (EP 0 654 464, WO 2011/162397, WO 2012/084812, WO 2015/197530).

Chiral aryl-substituted bicyclic pyridine-phosphinites can be prepared via resolution of racemic aryl-substituted 6,7-dihydro-5H-cyclopenta[b]pyridin-7-ols using chiral HPLC and conversion to the corresponding phosphinites by deprotonation and subsequent treatment with di(cyclo)alkyl chlorophosphane (S. Kaiser et al., Angew. Chem. Int. Ed. 2006, 45, 5194-5197). A kinetic resolution of the racemic alcohols with lipase or by copper-catalyzed benzoylation is also known (D. H. Woodmansee et al., Chem. Sci. 2010, 1, 72-78; C. Mazet et al., Org. Lett. 2006, 8, 1879-1882). However, a general disadvantage of racemic resolution methods is that the desired and undesired enantiomers are always obtained in equal amounts which requires additional steps to convert the undesired enantiomer to the desired enantiomer, e.g. by repeated oxidation and racemic resolution sequences.

In principle, methods for asymmetric reduction of bicyclic pyridine ketones are also already available. An asymmetric transferhydrogenation of bicyclic pyridine ketones in presence of a chiral iron catalyst is known from A. Naik et al., Chem. Commun. 2010, 46, 4475-4477. However, it was found that a substituent in 2-position is detrimental to the enantioselectivity, and accordingly 2-aryl substituted bicyclic pyridine alcohols could only be obtained with moderate enantioselectivities (52-72% *ee).* Similarly, a catalytic enantioselective reduction of 2-phenyl-6,7-dihydro-5H-quinolin-8-one with (S)-Me-CBS-borane was reported to afford the corresponding ketone with an enantiomeric excess of 72% only (Tetrahedron: Asymmetry, 2009, 20, 1425-1432).

In the light of the prior art described above, it is an object of the present invention to provide a process for preparing optically active 2-aryl substituted 6,7-dihydro-5H-cyclopenta[b]pyridin-7-ol derivatives which process has advantages over the processes of the prior art. In particular, the process should allow the desired enantiomer to be prepared in high yield and high enantiomeric purity.

The object described above was achieved by a process for preparing a compound of the formula **(Ia)** or **(Ib),**

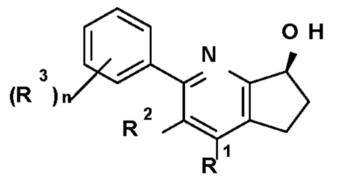

**(Ia)**

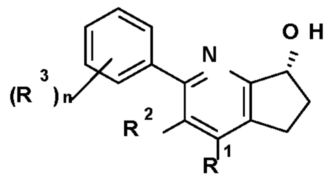

**(Ib)**

where

| | |
|---|---|
| $R^1$ and $R^2$ | are independently from one another selected from the group consisting of hydrogen and $C_1$-$C_4$-alkyl, |
| each $R^3$, | if present, is independently selected from $C_1$-$C_4$-alkyl, and |
| n | is 0, 1, 2 or 3, |

comprising asymmetric transfer hydrogenation of a ketone of the formula **(II)**

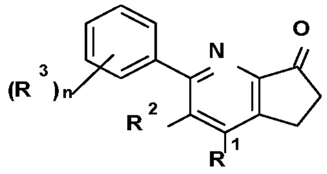

**(II)**

in which the substituents $R^1$, $R^2$, $R^3$ and the integer n are as defined for the compound of the formula **(Ia)** or **(Ib),**

in presence of a chiral ruthenium catalyst and a polar solvent, wherein the ruthenium catalyst has the general formula **(Va), (Vb), (VIa)** or **(VIb)** shown below.

It has been found, surprisingly, that optically active 2-aryl-substituted 6,7-dihydro-5H-cyclopenta[b]pyridin-7-ol derivatives (of the formulae **Ia** and **Ib)** can be prepared in high yields and excellent enantioselectivity by asymmetric transfer hydrogenation of the corresponding 2-aryl substituted bicyclic pyridine ketones (of the formula **II**) in presence of a chiral ruthenium catalyst of formula **(Va), (Vb), (VIa)** or **(VIb)** shown below.

*Definitions*

In the definitions of the symbols given in the formulae above and below, collective terms were used, which are generally representative of the following substituents:
The term "halogen" as used herein refers to fluorine, chlorine, bromine or iodine atom.

The term "$C_1$-$C_4$-alkyl" as used herein refers to a saturated, branched or straight hydrocarbon chain having 1, 2, 3 or 4 carbon atoms. Examples of $C_1$-$C_4$-alkyl include methyl, ethyl, propyl (n-propyl), 1-methylethyl (*iso*-propyl), butyl (n-butyl), 1-methylpropyl (*sec*-butyl), 2-methylpropyl (iso-butyl) and 1,1-dimethylethyl *(tert*-butyl).

The term "$C_2$-$C_6$-alkyl" as used herein refers to a saturated, branched or straight hydrocarbon chain having 2, 3, 4, 5 or 6 carbon atoms. Examples of $C_2$-$C_6$-alkyl include but are not limited to ethyl, propyl (n-propyl), 1-methylethyl (iso-propyl), butyl (n-butyl), 1-methylpropyl (sec-butyl), 2-methylpropyl (iso-butyl), 1,1-dimethylethyl (tert-butyl), pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl.

The terms "phenyl-$(CH_2)_3$-", "phenyl-$(CH_2)_4$-" and "phenyl-$(CH_2)_2$-O-$CH_2$-" as used herein refer to a phenyl group, which is unsubstituted or substituted as defined herein and which is attached to the parent moiety via a -$(CH_2)_3$-, -$(CH_2)_4$- or -$(CH_2)_2$-O-$CH_2$- linker.

As used herein, when a group is said to be "substituted", the group may be substituted with one or more substituents where the substituents may be identical or different. The expression "one or more substituents" refers to a number of substituents that ranges from one to the maximum number of substituents possible based on the number of available bonding sites, provided that the conditions of stability and chemical feasibility are met.

The term "enantioselective" as used herein means that one of the two possible enantiomers of the hydrogenation product, namely the enantiomer of the formula **(Ia)** (or **(Ia')**) or the enantiomer of the formula **(Ib)** (or **(Ib')),** is preferably formed. The "enantiomeric excess" or *"ee"* indicates the degree of enantioselectivity:

$$\% \, ee = \frac{major\ enantiomer\ (mol) - minor\ enantiomer\ (mol)}{major\ enantiomer\ (mol) + minor\ enantiomer\ (mol)} x\ 100\%$$

The major enantiomer can be controlled by the selection of the chiral ligand, for example by selecting the chiral ligand of the formula (**IIIa**) or the opposite enantiomer (the ligand of the formula (**IIIb**)), or by selecting the chiral ligand of the formula **(IVa)** or the opposite enantiomer (the ligand of the formula **(IVb)).**

The process according to the invention is used for preparing the compound of the formula **(Ia)** or **(Ib),** preferably **(Ia),** using a compound of the formula (**II**) as starting material.

Preferred are compounds of the formulae **(Ia), (Ib)** and **(II),** in which $R^2$ is H.

More preferred compounds of the formula **(Ia), (Ib)** and (**II**) are compounds of formulae **(Ia'), (Ib')** and (**II'**)

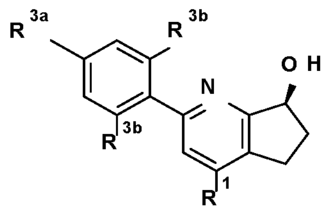

(Ia')

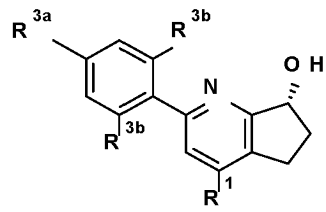

(Ib')

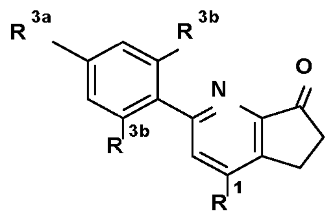

(II'),

wherein $R^1$, $R^{3a}$and $R^{3b}$ are independently of one another selected from $C_1$-$C_4$-alkyl.

Even more preferred are compounds of the formulae **(Ia), (Ib)** and (**II**) and compounds of the formulae (**Ia'**), **(Ib')** and (**II'**), in which $R^1$ is methyl.

Particularly preferred are compounds of the formulae **(Ia'), (Ib')** and **(II'),** in which

$R^1$ is methyl,

$R^{3a}$ is methyl, and

$R^{3b}$ is ethyl.

The process according to the invention comprises asymmetric transfer hydrogenation of the compound of the formula (**II**), preferably (**II'**). The substituents $R^1$, $R^2$ and $R^3$ and the integer n in the compound of the formula (**II**) are each as defined for the compounds of the formulae (Ia) and (Ib). Accordingly, the substituents $R^1$, $R^{3a}$ and $R^{3b}$ in the compound of the formula (II') are each as defined for the compounds of the formulae (**Ia'**) and (Ib').

The asymmetric transfer hydrogenation of the compound of the formula (II) is conducted in the presence of a chiral ruthenium catalyst of general formula (Va), (Vb), (VIa) or (VIb):

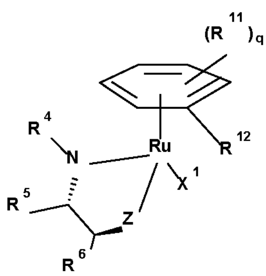

(Va)

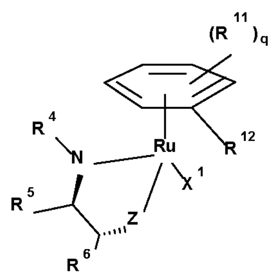

(Vb)

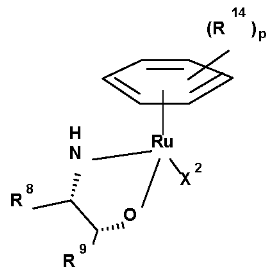

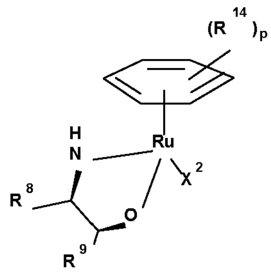

**(VIa)** **(VIb)**

wherein

Z is $NR^{13}$ or O,

$R^4$ is phenylsulphonyl, wherein the phenyl is unsubstituted or substituted with one or more substituents independently selected from $C_1$-$C_4$-alkyl and halogen, or

$R^4$ is 2-pyrrolidinylcarbonyl, preferably (*2S*)-2-pyrrolidinylcarbonyl,

$R^5$ and $R^6$ together form a $-(CH_2)_4-$ group, or

$R^5$ and $R^6$ are unsubstituted phenyl,

each $R^{11}$, if present, is independently selected from $C_1$-$C_4$-alkyl,

$R^{12}$ is $C_1$-$C_4$-alkyl or hydrogen and

$R^{13}$ is hydrogen, or

$R^{12}$ and $R^{13}$ form together a $-(CH_2)_3-$, $-(CH_2)_4-$, $-CH_2-O-CH_2-$, $*-(CH_2)_2-O-CH_2-\#$ or $*-(CH_2)-O-(CH_2)_2-\#$ group, where the bond marked with "*" is bonded to the nitrogen and the bond marked with "#" is bonded to the phenyl ring,

q is 0, 1, 2, 3, 4 or 5,

$X^1$ is chlorine or bromine, or

$X^1$ is $BF_4^-$, $PF_6^-$ or $SbF_6^-$, in which case the Ru-$X^1$ bond is of a coordinative or ionic nature and the Ru has a positive charge,

$R^8$ and $R^9$ are unsubstituted phenyl, or

$R^8$ and $R^9$ form together a group of the formula

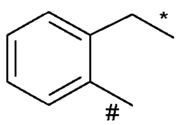

,

wherein the bond identified by "*" is connected to the carbon bearing the hydroxyl group and the bond identified by "#" is connected to the carbon bearing the amino group, each $R^{14}$, if present, is independently selected from $C_1$-$C_4$-alkyl,

p is 0, 1, 2, 3, 4, 5 or 6, and

$X^2$ is chlorine or bromine, or

$X^2$ is $BF_4^-$, $PF_6^-$ or $SbF_6^-$, in which case the Ru-$X^2$ bond is of a coordinative or ionic nature and the Ru has a positive charge.

Depending on whether compound **(Ia)** or **(Ib)** is the desired product, a catalyst of the formula **(Va)** or **(VIb)** or a catalyst of the formula **(Vb)** or **(VIa)** is suitable for use in the process according to the invention. Usually, if a compound of the formula **(Ia)** is the desired product, a catalyst of the formula **(Va)** or **(VIb),** preferably **(Va),** is suitable for use in the process according to the invention, whereas if a compound of the formula **(Ib)** is the desired product, a catalyst of the formula **(Vb)** or **(VIa),** preferably **(Vb),** is suitable for use in the process according to the invention.
Particularly preferred are chiral ruthenium catalysts of the general formula **(Va)** or **(Vb),** where

Z is $NR^{13}$ or O,

$R^4$ is phenylsulphonyl, wherein the phenyl is unsubstituted or substituted with one or more substituents independently selected from $C_1$-$C_4$-alkyl and fluorine,

$R^5$ and $R^6$ together form a -$(CH_2)_4$- group, or

$R^5$ and $R^6$ are unsubstituted phenyl, and

each $R^{11}$, if present, is methyl,

$R^{12}$ is $C_1$-$C_4$-alkyl, for example methyl or isopropyl, and

$R^{13}$ is hydrogen, or

$R^{12}$ and $R^{13}$ form together a -$(CH_2)_3$-, -$(CH_2)_4$-, -$CH_2$-O-$CH_2$-, *-$(CH_2)_2$-O-$CH_2$-# or *-$(CH_2)$-O-$(CH_2)_2$-# group, where the bond marked with "*" is bonded to the nitrogen and the bond marked with "#" is bonded to the phenyl ring,

q is 0, 1 or 2,

$X^1$ is chlorine or bromine, or

$X^1$ is $BF_4^-$, $PF_6^-$ or $SbF_6^-$, in which case the Ru-$X^1$ bond is of a coordinative or ionic nature and the Ru has a positive charge.

Chiral ruthenium catalysts of the formulae **(Va), (Vb), (VIa)** and **(VIb)** are commercially available or may be prepared by methods known in the art (e.g. R. Hodgkinson et al., Organometallics, 2014, 33, 5517-5524; V. Parekh et al., Catal. Sci. & Technol., 2012, 2, 406-414).
Chiral ruthenium catalysts of the formulae **(Va)** and **(Vb),** wherein $R^{12}$ is $C_1$-$C_4$-alkyl and Z is O or NH, and chiral ruthenium catalysts of the formulae **(VIa)** and **(VIb)** may be formed *in situ* by mixing a dichloro (aromatic ligand)ruthenium (II) dimer precatalyst, such as $[RuCl_2(p\text{-cymene})]_2$ or $[RuCl_2(\text{hexamethylbenzene}]_2$, or a dibromo (aromatic ligand) ruthenium (II) dimer precatalyst with a chiral ligand of the formula **(IIIa’),** (**IIIb’**), **(IVa)** or **(IVb),**

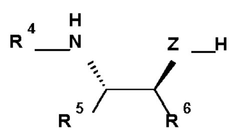

**(IIIa’)**

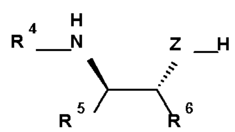

**(IIIb’)**

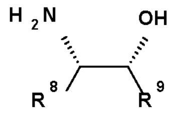 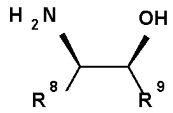

**(IVa)** **(IVb),**

wherein

$R^4$, $R^5$ and $R^6$ are each as defined for the complexes of the formulae **(Va)** and **(Vb),**

Z is NH or O,

$R^8$ and $R^9$ are each as defined for the complexes of the formulae **(VIa)** and **(VIb),**

and wherein the aromatic ligand of the precatalyst is selected from the group consisting of *p*-cymene and benzene, which is optionally substituted with one or more methyl groups,

in an organic solvent (e.g. dichloroethane).

Examples of suitable organic solvents are dichloromethane, 1,2-dichloroethane, chlorobenzene, dichlorobenzene, toluene, acetonitrile, dimethylformamide, ethanol, isopropanol, tetrahydrofuran and 2-methyl tetrahydrofurane.
Examples of suitable aromatic ligands are *p*-cymene and hexamethylbenzene.
The amount of ruthenium catalyst used is preferably within the range of from 0.01 mol% to 10 mol%, more preferably 0.1 mol% to 5 mol%, and most preferably 0.5 mol% to 3 mol%, based on the amount of the compound of the formula **(II).**
The process according to the invention comprises asymmetric transfer hydrogenation of the compound of the formula **(II).**
The hydrogen source used is preferably selected from the group consisting of sodium formate, potassium formate, lithium formate, calcium formate, magnesium formate, formic acid/triethylamine, potassium *tert*-butylate/isopropanol, sodium tert-butylate/isopropanol and lithium tert-butylate/isopropanol, more preferably selected from the group consisting of sodium formate, potassium formate, lithium formate, calcium formate, magnesium formate and formic acid/triethylamine, and most preferably selected from sodium formate and formic acid/triethylamine.
The amount of hydrogen source used is preferably at least 1.0 equivalent, more preferably at least 2.0 equivalents, and most preferably 2.0 to 3.5 equivalents, based on the amount of the compound of the formula **(II).**
In case of formic acid/triethylamine, the formic acid acts as hydrogen source and the amount of hydrogen source used therefore corresponds to the amount of formic acid used. Preferably, the amount of triethylamine used is within the range of from 0.2 to 1.0 equivalents, based on the amount of the compound of the formula **(II).**
In case of potassium *tert*-butylate/isopropanol, sodium *tert*-butylate/isopropanol and lithium *tert*-butylate/isopropanol, the isopropanol acts both as a hydrogen source and as a (co-)solvent and the amount of isopropanol used is therefore typically significantly more than the amount of hydrogen source required for the hydrogenation reaction. The amount of *tert*-butylate used is preferably between 0.2 and 1.0 equivalents, based on the amount of the compound of the formula **(II).**
Particularly preferably, the hydrogen source used is selected from sodium formate and formic acid/triethylamine and the amount of hydrogen source used is within the range of from 2.0 to 3.5 equivalents, based on the amount of the compound of the formula **(II).**
The transfer hydrogenation is preferably conducted at a temperature within the range of from 10 °C to 100 °C, more preferably 20 °C to 80 °C, and in particular 25°C to 50°C.
The reaction time is not critical and may, according to the batch size, be selected within a relatively wide range. Typical reaction times are between 30 min and 24 h.
According to the invention, the asymmetric transfer hydrogenation of the compound of the formula **(II)** is conducted in the presence of a polar solvent.
Suitable polar solvents are selected from the group consisting of dichloromethane, methanol, ethanol, isopropanol, *n*-butanol, tetrahydrofuran, 2-methyl-tetrahydrofuran, dimethylformamide, acetonitrile, methanol/water, ethanol/water, isopropanol/water, *n*-butanol/water, tetrahydrofuran/water, 2-methyl-tetrahydrofuran/water, dimethylformamide/water, acetonitrile/ water, and mixtures thereof.
Preferred polar solvents are selected from the group consisting of ethanol, isopropanol, 2-methyl-tetrahydrofuran, dimethylformamide, acetonitrile, ethanol/water, isopropanol/water, 2-methyl-tetrahydrofuran/water, dimethylformamide/water, acetonitrile/ water, and mixtures thereof.

Particularly preferred are ethanol, isopropanol/water, dimethylformamide/water, acetonitrile/water; 2-methyltetrahydrofuran and 2-methyl-tetrahydrofuran/water.

If the transfer hydrogenation is conducted in presence of water, the work-up and isolation of the compound of formula **(Ia)** or **(Ib)** can be effected by the following steps: (i) separating the aqueous phase from the organic phase, (ii) extracting the aqueous phase one or more times with a suitable organic solvent (e.g. heptane, toluene or xylene), (iii) washing the combined organic phases with water, brine and/or aqueous sodium bicarbonate solution, (iv) drying the obtained organic phase by treatment with magnesium sulfate or via azeotropic distillation and (v) removing (part of) the organic solvent by distillation. The obtained product may be purified by crystallization from heptane.

The compounds of formula **(Ia)** or **(Ib)** can be prepared in high enantioselectivity by means of the asymmetric transfer hydrogenation according to the invention. The compound of formula **(Ia)** or **(Ib)** obtained by the process according to the invention can be purified by forming a crystalline addition salt with camphor sulfonic acid. This can increase the chemical purity of the desired product to > 99%w/w.

The process according to the invention comprises asymmetric transfer hydrogenation of the compound of the formula **(II).**

Ketones of the formula **(II)** may be obtained from a racemic mixture of the compounds of formulae **(Ia)** and **(Ib)**

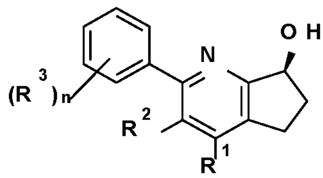

**(Ia)**

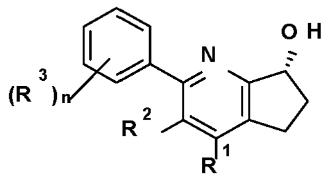

**(Ib),**

wherein the substituents $R^1$, $R^2$, $R^3$ and the integer n are each as defined for the compound of the formula (**II**), by oxidation using TEMPO, a TEMPO derivative or a TEMPO analogue, a hypochlorite salt and optionally a bromide salt.

Analogously, ketones of the formula (**II'**) may be obtained from a racemic mixture of the compounds of the formulae **(Ia')** and **(Ib')**

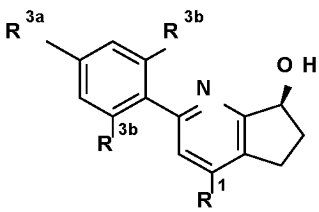

**(Ia')**

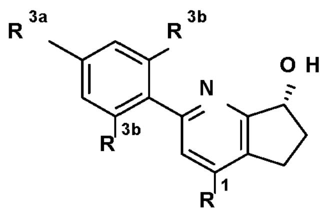

**(Ib')**

wherein the substituents $R^1$, $R^{3a}$ and $R^{3b}$ are each as defined for the compound of the formula **(II'),**
by oxidation using TEMPO, a TEMPO derivative or a TEMPO analogue, a hypochlorite salt and optionally a bromide salt.

It has been found that the ketone of formula **(II)** may be obtained from a racemic mixture of the compounds of formulae **(Ia)** and **(Ib)** by a TEMPO-mediated oxidation using catalytic amounts of TEMPO, a TEMPO derivative or a TEMPO analogue, a hypochlorite salt as oxidation agent and optionally bromide as co-oxidation agent. It has been found that this reaction works well with compounds of the formula **(Ia)** and **(Ib)** having a pyridine functionality, which is surprising in light of previous results disclosed in M. Shibuya, M Tomizawa, I. Suzuki, Y. Iwabuchi, J. Am. Chem. Soc., 2006, 128, 8412-8413. Shibuyaet al. teach that nitrosyl radicals, such as TEMPO and 1-Me-AZADO, do not efficiently oxidize substrates containing a basic nitrogen. Moreover, the reaction also works well using catalytic amounds of readily available TEMPO, which is suprising because Shibuya et al. teach to use 1-Me-AZADO (2-aza-1-methyladamantane *N*-oxyl) instead of TEMPO for the oxidation of secondary alcohols.

Examples of suitable TEMPO derivatives and TEMPO analogues are 4-hydroxy-TEMPO, 4-methoxy-TEMPO, 4-oxo-TEMPO, 2-azaadamantane *N-oxyl* and 2-aza-1-methyladamantane *N-oxyl.*

The TEMPO, TEMPO derivative or TEMPO analogue may be used as such or in immobilized form. Suitable examples of immobilized TEMPO are silica-supported TEMPO and polystyrene-supported TEMPO.

The amount of TEMPO, TEMPO derivative or TEMPO analogue used is preferably within the range of from 0.5 mol% to 20 mol%, more preferably 1 mol% to 10 mol%, most preferably 3 mol% to 7.5 mol%, based on the total amount of the compounds of formulae **(Ia)** and **(Ib),** preferably **(Ia')** and **(Ib').**

Suitable hypochlorite salts are sodium hypochlorite, potassium hypochlorite and magnesium hypochlorite. Preferably, the hypochlorite salt used in the TEMPO-mediated oxidation is selected from sodium hypochlorite and potassium hypochlorite. Sodium hypochlorite is particularly preferred.

The amount of hypochlorite salt used is preferably within the range of from 1 to 5 equivalents, more preferably 1.1 to 2.0 equivalents, most preferably 1.2 to 1.5 equivalents, based on the total amount of the compounds of the formulae **(Ia)** and **(Ib),** preferably **(Ia')** and (**Ib'**).

Suitable bromide salts are potassium bromide, sodium bromide and tetrabutylammonium bromide, and mixtures thereof.

The amount of bromide salt used is preferably within the range of from 0.5 mol% to 20 mol%, more preferably 5 mol% to 15 mol%, based on the total amount of the compounds of the formulae **(Ia)** and **(Ib),** preferably **(Ia')** and **(Ib').**

The TEMPO-mediated oxidation is preferably conducted under basic two-phase conditions in presence of water, an organic solvent and a phase-transfer catalyst, such as tetrabutylammoniumbromide (TBAB).

Suitable organic solvents are selected from the group consisting of dichloromethane, 1,2-dichloroethane, chlorobenzene, dichlorobenzene, toluene, acetonitrile, ethyl acetate, *n*-propyl acetate, *n*-butyl acetate and similar solvents that are inert towards oxidation by hypochlorite reagents.

For example, the TEMPO-mediated oxidation may be conducted under basic two-phase conditions using a mixture of an aqueous sodium hypochlorite solution and a saturated aqueous solution of sodium bicarbonate as aqueous phase, dichloromethane as organic solvent and tetrabutylammoniumbromide (TBAB) as phase-transfer catalyst.

The TEMPO-mediated oxidation is preferably conducted at a temperature within the range of from -20 °C to +25 °C, preferably -5°C to +5°C.

The reaction time is not critical and may, according to the batch size, be selected within a relatively wide range. Typical reaction times are between 5 min and 3 h.

The work-up and isolation of the ketone of formula **(II)** or **(II')** may be effected by the following steps: (i) separating the aqueous phase from the organic phase, (ii) extracting the aqueous phase one or more times with a suitable organic solvent (e.g. heptane), (iii) washing the combined organic phases with water or brine, (iv) drying the obtained organic phase by treatment with magnesium sulfate and (v) removing the organic solvent by distillation. The obtained product of formula **(II)** or (**II'**) may be purified by crystallization from heptane.

**Abbreviations and Acronyms:**

| | |
|---|---|
| % a/a | area percentage |
| DCE | 1,2-dichloroethane |
| DMF | dimethylformamide |
| *ee* | enantiomeric excess |
| Et | ethyl |
| EtOH | ethanol |
| HPLC | high performance liquid chromatography |
| Ph | phenyl |
| $^{i}$PrOH | isopropanol |
| qNMR | quantitative NMR |
| Me | methyl |
| MeCN | acetonitrile |
| MeTHF | 2-methyl tetrahydrofurane |
| $NEt_3$ | triethylamine |
| NMR | nuclear magnetic resonance |
| TEMPO | 2,2,6,6-tetramethylpiperidinyloxyl |
| TMB | trimethoxybenzene |
| Ts | tosyl (p-toluenesulfonyl) |

## Examples

### Preparation of the starting material (II'-1) by TEMPO-mediated oxidation of (Ia'-1)/(Ib'-1):

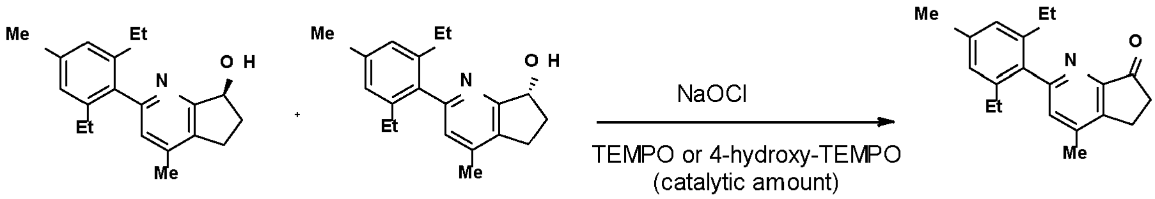


(**Ia'-1**) (**Ib'-1**) (**II'-1**)

Example 1 :

A racemic mixture of compounds **(Ia'-1) and (Ib'-1)** (93.3% w/w, 1421.5 g, 4489 mmol), TEMPO (35 g, 224 mmol), potassium bromide (53 g, 449 mmol), tetrabutylammonium bromide (72 g, 224 mmol), dichloromethane (6.8 L) and saturated sodium bicarbonate solution (made with 4.5L of water) were placed in a reactor. The beige colored mixture was cooled down to 0°C and a mixture of sodium hypochlorite solution (13.4% w/w, total amount needed: 3530 g, 5454 mmol, 1.215 equiv) and saturated sodium bicarbonate solution (total amount needed: 3.81 kg) was added at 0°C (± 4°C) under temperature control until in-process control (HPLC@220nm) showed complete conversion of starting material. The reaction mixture was transferred into a stirring vessel and was diluted with water (2.8 L). The aqueous phase was separated and re-extracted with dichloromethane (5.6 L). The combined organic layers were washed with water (5.6 L) and filtered through a sodium sulfate plug (1 kg), which was rinsed with dichloromethane (2.8 L). 10 L of solvent were distilled off(40 °C) and 6 L of heptane were added. Again 4.5 L of solvent were distilled off and replaced by heptane. 1 L of heptane was distilled off and the solution was seeded by adding 2 g of compound **(II'-1)** to initiate crystallization. The suspension was concentrated at 45°C to a total mass of 8 kg, cooled down and rotated at 0-5°C for 3 hours. The solid was filtered off and washed with cold heptane (5 L, 0 - 5°C). The solid was dried under vacuum at 40-45°C.

Mass: 1273 g (97% of theory); Appearance: beige solid; HPLC (220nm): ≥ 99% area; Assay (1H-NMR, DMSO-d6, TMB as Standard): 96%. Yield (mass yield x assay): 93% of compound **(II'-1).**

Example 2

A racemic mixture of compounds **(Ia'-1)** and **(Ib'-1)** (0.13 g, 0.44 mmol), 4-hydroxy-TEMPO (3.8 mg, 0.022 mmol), potassium bromide (15 mg, 0.044 mol), tetrabutylammonium bromide (7.1 mg, 0.022 mmol), dichloromethane (2.6 mL) and saturated sodium bicarbonate solution (1.3 mL) were placed in a vial under inert atmosphere ($N_2$). The beige colored mixture was cooled down to 0°C and a mixture of sodium hypochlorite solution (10-14%w/w, 0.7 mL) and saturated sodium bicarbonate solution (0.9 mL) was added dropwise at 0°C (± 4°C) during 5 min. After 20 min stirring at this temperature, in-process control (HPLC@220nm) showed complete conversion of starting material and 82.9% a/a of compound (**II'-1**).

Example 3

A racemic mixture of compounds **(Ia'-1) and (Ib'-1)** (0.13 g, 0.44 mmol), silica-supported TEMPO (0.35 mmol TEMPO per gramm of material, 63 mg, 0.022 mmol), potassium bromide (15 mg, 0.044 mol), tetrabutylammonium bromide (7.1 mg, 0.022 mmol), dichloromethane (2.6 mL) and saturated sodium bicarbonate solution (1.3 mL) were placed in a vial under inert atmosphere ($N_2$). The beige colored mixture was cooled down to 0°C and a mixture of sodium hypochlorite solution (10-14%w/w, 0.7 mL) and saturated sodium bicarbonate solution (0.9 mL) was added dropwise at 0°C (± 4°C) during 5 min. After 20 min stirring at this temperature, in-process control (HPLC@220nm) showed complete conversion of starting material and 96.6% a/a of compound **(II'-1).**

Example 4

A racemic mixture of compounds **(Ia'-1) and (Ib'-1)** (0.13 g, 0.44 mmol), polystyrene-supported TEMPO (1 mmol TEMPO per gramm of material, 22 mg, 0.022 mmol), potassium bromide (15 mg, 0.044 mol), tetrabutylammonium

bromide (7.1 mg, 0.022 mmol), dichloromethane (2.6 mL) and saturated sodium bicarbonate solution (1.3 mL) were placed in a vial under inert atmosphere ($N_2$). The beige colored mixture was cooled down to 0°C and a mixture of sodium hypochlorite solution (10-14% w/w, 0.7 mL) and saturated sodium bicarbonate solution (0.9 mL) was added dropwise at 0°C (± 4°C) during 5 min. After 20 min stirring at this temperature, in-process control (HPLC@220nm) showed complete conversion of starting material and 90.4%ala of compound **(II'-1).**

## Asymmetric Transferhydrogenation

Reactions were performed in glass vessels of appropriate dimensions. Unless stated otherwise, reaction mixtures were analyzed without workup via HPLC (Chiralpak IC column, heptane/ethanol gradient (with 0.02% of diethylamine as stabilizing additive), 1 mL/min).

*Preparation of chiral ruthenium catalysts*

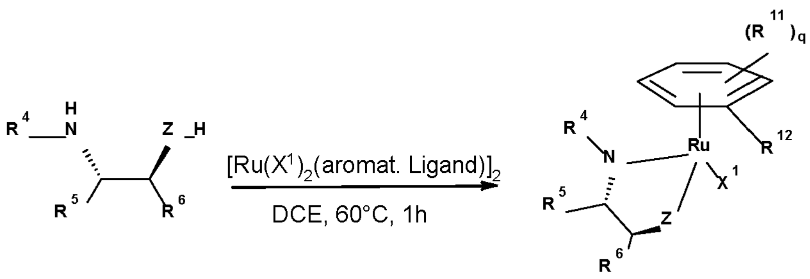


(**IIIa'**) (**Va**)

aromat. Ligand:
p-cymene ($R^{12}$ = isopropyl, $R^{11}$ = methyl, q = 1) or
hexamethylbenzene ($R^{11}$, $R^{12}$ = methyl, q = 5)

$X^1$ = Cl

analogously:

(**IIIb'**) [Ru($X^1$)$_2$(aromat. Ligand)]$_2$ DCE, 60°C, 1h (**Vb**)

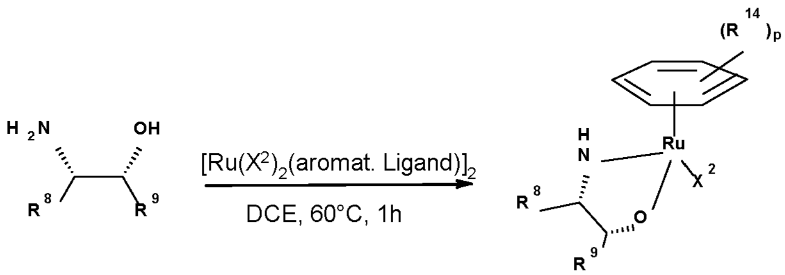


(**IVa**) (**VIa**)

aromat. Ligand:
p-cymene ($R^{14}$ = isopropyl, methyl, p = 2) or
hexamethylbenzene ($R^{14}$ = methyl, p = 6)

$X^2$ = Cl

The catalysts used in examples 5-14 were preformed prior to reaction by dissolving a ruthenium (II) catalyst precursor ([$RuCl_2$(*p*-cymene)]$_2$ or [$RuCl_2$(hexamethylbenzene]$_2$, 1.0 equiv.) in DCE at 60 °C, adding the ligand given in table 1 (1.2 equiv.) and stirring of the solution for 1 h at 60°C, followed by evaporation of DCE.

The following catalysts are commercially available and were used as purchased in examples 15-34:

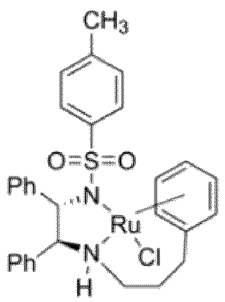

(Va-1)

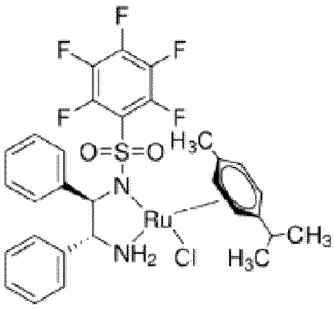

(Vb-2)

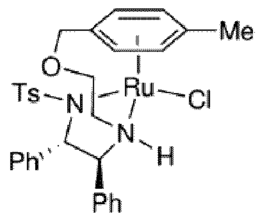

(Va-3)

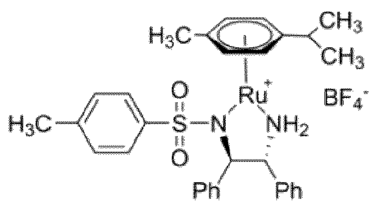

(Vb-4)

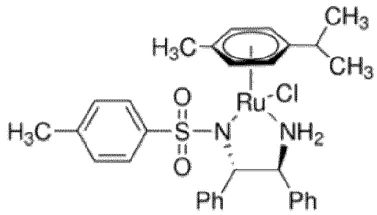

(Va-5)

*Transfer hydrogenation reaction*

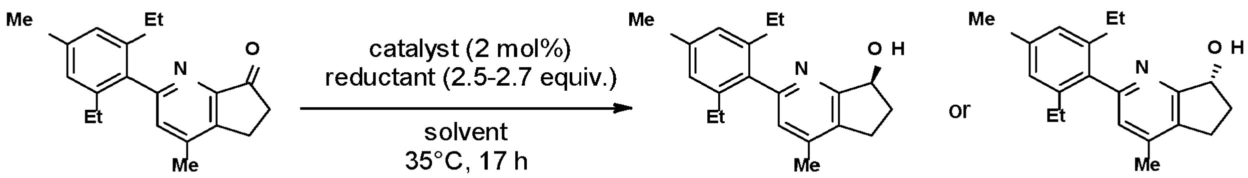


(II'-1) (Ia'-1) (Ib'-1)

Under an inert gas atmosphere, one well of a 96 well-plate autoclave was filled with 9.7 mg of ketone starting material (II'-1) (33 µmol, 1 equiv), reductant (see table 1; $NaCO_2H$: 2.5 equiv.; $HCO_2H/NEt_3$: 2.7 equiv./0.6 equiv., respectively), 0.66 µmol of catalyst (2 mol%, see table 1) in the respective solvent mixture (see table 1, starting material concentration is 0.13 M). The autoclave was closed and heated to 35 °C and the reaction mixture was shaken at that temperature for 17 h. Chromatographic analysis of the cooled and de-pressurized reaction mixture showed the %a/a HPLC conversion rates of starting material (II'-1) to the reduced alcohol product **(Ia'-1)** or **(Ib'-1).** The %alaHPLC conversion rates and enantioselectivities are depicted in table 1 below.

Table 1:

| Ex. | Catalyst Precursor[1)] / Catalyst[2)] | Ligand | Solvent | Reductant | Product (major enantiomer, | Conversion (%a/a HPLC) | %ee |
|---|---|---|---|---|---|---|---|
| 5* | [$RuCl_2$(p-cymene)]$_2$ | 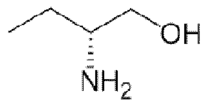 | $^i$PrOH; $H_2O$ | $HCO_2Na$ | (**Ib'-1**) | 79.9 | 56.4 |
| 6 | [$RuCl_2$(p-cymene)]$_2$ | 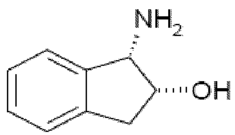 | DMF; $H_2O$ | $HCO_2Na$ | (**Ib'-1**) | 43.9 | 80.3 |
| 7 | [$RuCl_2$(p-cymene)]$_2$ | 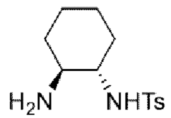 | $^i$PrOH; $H_2O$ | $HCO_2Na$ | (**Ia'-1**) | 100 | 92.3 |
| 8 | [$RuCl_2$(hexamethylbenzene)]$_2$ | 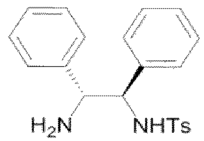 | MeTHF; $H_2O$ | $HCO_2Na$ | (**Ib'-1**) | 100 | 97.9 |
| 9 | [$RuCl_2$(p-cymene)]$_2$ | 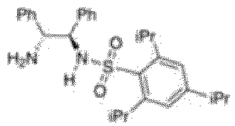 | MeCN; $H_2O$ | $HCO_2Na$ | (**Ib'-1**) | 100 | 96.1 |
| 10 | [$RuCl_2$(p-cymene)]$_2$ | 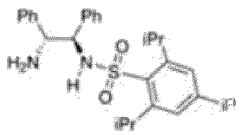 | DMF | $HCO_2H$; $NEt_3$ | (**Ib'-1**) | 86.9 | 89.1 |
| 11 | [$RuCl_2$(p-cymene)]$_2$ | 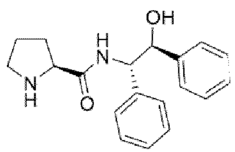 | DMF; $H_2O$ | $HCO_2Na$ | (**Ib'-1**) | 100 | 96.8 |

(continued)

| Ex. | Catalyst Precursor1) / Catalyst2) | Ligand | Solvent | Reductant | Product (major enantiomer, | Conversion (%a/a HPLC) | %ee |
|---|---|---|---|---|---|---|---|
| 12 | $[RuCl_2(p\text{-cymene})]_2$ | 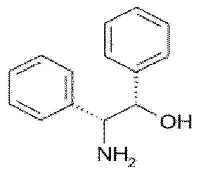 | MeTHF; $H_2O$ | $HCO_2Na$ | (**Ia'-1**) | 97.2 | 84 |
| 13* | $[RuCl_2(hexamethylbenzene)]_2$ | 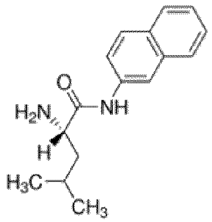 | MeCN; $H_2O$ | $HCO_2Na$ | (**Ib'-1**) | 98.4 | 30.4 |
| 14* | $[RuCl_2(p\text{-cymene})]_2$ | 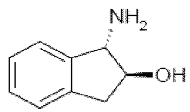 | EtOH; $H_2O$ | $HCO_2Na$ | (**Ib'-1**) | 21.8 | 16.7 |
| 15 | (**Va-1**) | - | EtOH | $HCO_2H$; $NEt_3$ | (**Ia'-1**) | 100 | 97.5 |
| 16 | (**Va-1**) | - | DMF; $H_2O$ | $HCO_2Na$ | (**Ia'-1**) | 98.9 | 95.9 |
| 17 | (**Va-1**) | - | MeTHF | $HCO_2H$; $NEt_3$ | (**Ia'-1**) | 83.0 | 93.6 |
| 18 | (**Va-1**) | - | MeCN; $H_2O$ | $HCO_2Na$ | (**Ia'-1**) | 99.1 | 97.3 |
| 19 | (**Vb-2**) | - | EtOH | $HCO_2H$; $NEt_3$ | (**Ib'-1**) | 100 | 97.1 |
| 20 | (**Vb-2**) | - | DMF; $H_2O$ | $HCO_2Na$ | (**Ib'-1**) | 100 | 94.5 |
| 21 | (**Vb-2**) | - | MeTHF | $HCO_2H$; $NEt_3$ | (**Ib'-1**) | 100 | 95.5 |
| 22 | (**Vb-2**) | - | MeCN; $H_2O$ | $HCO_2Na$ | (**Ib'-1**) | 100 | 96.9 |
| 23 | (**Va-3**) | - | EtOH | $HCO_2H$; $NEt_3$ | (**Ia'-1**) | 100 | 92.1 |
| 24 | (**Va-3**) | - | DMF; $H_2O$ | $HCO_2Na$ | (**Ia'-1**) | 98.5 | 98.5 |
| 25 | (**Va-3**) | - | MeTHF | $HCO_2H$; $NEt_3$ | (**Ia'-1**) | 96.6 | 91.6 |
| 26 | (**Va-3**) | - | MeCN; $H_2O$ | $HCO_2Na$ | (**Ia'-1**) | 100 | 98.7 |
| 27 | (**Vb-4**) | - | EtOH | $HCO_2H$; $NEt_3$ | (**Ib'-1**) | 100 | 96.2 |

(continued)

| | | | | | | | |
|---|---|---|---|---|---|---|---|
| 13* | $[RuCl_2(hexamethylbenzene)]_2$ | 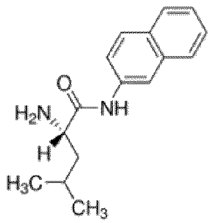 | MeCN; $H_2O$ | $HCO_2Na$ | (**Ib'-1**) | 98.4 | 30.4 |
| 28 | (**Vb-4**) | - | MeTHF | $HCO_2H$; $NEt_3$ | (**Ib'-1**) | 100 | 96.6 |
| 29 | (**Vb-4**) | - | DMF; $H_2O$ | $HCO_2Na$ | (**Ib'-1**) | 98.2 | 96.3 |
| 30 | (**Va-5**) | - | EtOH | $HCO_2H$; $NEt_3$ | (**Ia'-1**) | 100 | 94.1 |
| 31 | (**Va-5**) | - | MeTHF | $HCO_2H$; $NEt_3$ | (**Ia'-1**) | 100 | 91.9 |
| 32 | (**Va-5**) | - | DMF; $H_2O$ | $HCO_2Na$ | (**Ia'-1**) | 98.9 | 96.7 |

1): The catalysts used in examples 5-12 are complexes of the formula **(Va), (Vb)** or **(VIa).** These catalysts were preformed prior to the reaction from the catalyst precursors and ligands given in the table according to the method described above. The catalysts used in examples 13 and 14 were prepared accordingly.
2): The catalysts used in examples 15-32 are commercially available and were employed as purchased.
*: Examples 5, 13, and 14, are not according to the invention and are present for comparison purposes only.

Example 33:

All solvents and solutions for the reaction and work-up procedure were degassed with argon before use. Ketone starting material **(II'-1)** (9.4 g, 32 mmol) and ethanol (70 ml) were placed in a 50ml three necked round bottom flask under argon atmosphere. Argon was bubbled through the suspension for 15 minutes before catalyst (Va-3) (2 mol%, 379 mg, 0.64 mmol) was added. A solution of sodium formate (24 g, 352 mmol) in water (94 ml) was added. The reaction was stirred at 35 °C (bath temperature) overnight (16 h). In a separation funnel, the oily upper layer was separated and the aqueous phase was extracted with heptane (50ml). The combined upper layers were diluted with heptane (25 ml) and washed with water (50 ml). The separated aqueous phase was re-extracted with heptane (40 ml). The combined organic phases were washed with water (50 ml) and brine (aqueous, 30%, 30 ml).

*Purification by Silica plug filtration*

A column was filled with silica gel 60 (Fluka 89943, 50g) as slurry in heptane. The organic layer from the extraction was directly applied on the column and eluted with a gradient from heptane (100%) to heptane / MeTHF 3 / 1 (v/v). Product fractions were evaporated in vacuum yielding 9.3 g of beige/brown solid (Assay: 96% w/w, 94% yield, 97% *ee).*

Example 34

The reaction was performed under inert gas atmosphere. All solvents and solutions for the reaction and work-up procedure were degassed with argon prior to use. Compound **(II'-1)** (1230g, 4025 mmol) and catalyst (**Va-3)** (54 g, 80 mmol) were placed in 20-L round bottom flask under inert gas atmosphere (Argon). Acetonitrile (4 L) was added and the mixture was mixed (30°C) to get a brownish to red solution **(Solution 1).** Sodium formate (1369 g, 20.1 mol) was dissolved in degassed water (7 L). The solution was three times evacuated and flushed with argon **(Solution 2). Solution 1** was placed in a reactor (flask rinsed with 0.5 L of acetonitrile) followed by **Solution 2** (flask rinsed with 1L of water).

The mixture was heated up to 35°C within about 45 minutes and stirred at this temperature for one hour. Process control showed full conversion of the starting material **(II'-1).** The reaction mixture was cooled down to 25°C and transferred into a separation vessel and the phases were separated. The aqueous layer was re-extracted with Heptane (3.7 L). The mixed organic phases (biphasic mixture) was washed with 2x 1.85 L half saturated aqueous sodium bicarbonate, followed by 1.85 L of saturated aqueous sodium bicarbonate solution. The organic layer was filtered over a sodium sulfate plug (800g) and rinsed with heptane (2x 1 L). The solvent was evaporated under vacuum (45°C) giving 1260 g of a brownish to violet resin.

Analytics: HPLC achiral(220nm): 97.6% area, HPLC chiral (220nm): *ee* 99.7%. The chemical yield was determined after purification via salt-formation and freebasing (cf. example 35).

*Camphersulfonic Acid Salt Formation*

Example 35:

Crude **(Ia'-1)** (from example 34, 1321 g) was dissolved in MeTHF (7 L) at 50°C. A solution of (1*S*)-(+)-10-camphor sulfonic acid (981g, 4221mmol) in MeTHF (4 L) was added continuously at 50°C within 20 minutes; the solution was seeded while adding. After complete addition, the formed suspension was stirred for additional 30 minutes at 50°C and then cooled down to 20°C within 1 hour. The solid was filtered off, washed with MeTHF (2x 1 L) and dried under vacuum at 45°C.

Yield: 1947 g (87% of theory) white solid, HPLC (220nm): ≥ 99% area

1945g of this material was dissolved in MeTHF (13 L) and water (5 L). 1.65 L of aqueous, saturated $Na_2CO_3$-solution was added to increase to pH to 10). The phases were separated, and the organic layer was washed with water (3.3 L) and brine (30%, 1.6 L). The organic layer was filtered over a sodium sulfate plug (1 kg) and rinsed with MeTHF (1.5 L). The solvent was evaporated in vacuum (45°C). The residue was co-evaporated with heptane (3x 1.3 L).

Yield: 1087g (beige solid). Analytics: 99.7% qNMR (DMSO-d6, internal standard: trimethoxybenzene); 99.7% ee (Chiralpak IC column, heptane/ethanol gradient (with 0.02% of diethylamine as stabilizing additive), 1 mL/min, 220nm). Purity corrected yield: 87% over 3 steps (transferhydrogenation (example 34), salt-formation, freebasing).

Example 36

The reaction was performed under inert gas atmosphere. All solvents and solutions for the reaction and work-up procedure were degassed with nitrogen prior to use. Compound (**II'-1**) (177 g, 589 mmol) and catalyst (**Va-3**) (1.92 g, 2.95 mmol, 0.5 mol%) were placed in a round bottom flask under inert gas atmosphere (Argon). Acetonitrile (646 mL) was added

and the mixture was mixed to get a brownish to red solution **(Solution** 1). Sodium formate (200.4 g, 2947 mmol) was dissolved in degassed water (1.15 L). The solution was further degassed by bubbling through nitrogen for 1 h **(Solution 2). Solution 2** was placed in a reactor followed by **Solution 1.**

The mixture was heated up to 35 °C within about 35 minutes and stirred at this temperature overnight. Process control showed full conversion of the starting material **(II'-1).** The reaction mixture was cooled down to 25 °C and transferred into a separation vessel and the phases were separated. From the organic layer, most of the acetonitrile is removed under reduced pressure (150-100 mbar) and a jacket temperature of 40 °C. The aqueous layer was re-extracted with xylene (233 g). The xylene layer is added to the distillation sump of the acetonitrile layer. Again, vacuum is applied (100 mbar, 50 °C jacket temperature), removing residues of acetonitrile, water, and part of the xylene (distillate amount: 233 g). A solution of (1*S*)-(+)-10-camphor sulfonic acid (136 g, 585 mmol) in MeTHF (420 g) was added continuously at 50 °C within 30 minutes. The mixture is kept at that temperature for 50 min, cooled to 10 °C within 2 h and then kept at 10 °C for a further 2 h. The mixture is filtered and washed twice with 226 g of MeTHF each. The filter cake is dried under vacuum at 30 °C yielding 288 g of Camphersulfonic acid salt in ≥99%a/a purity (92% yield (uncorrected) over two steps) and with an ee of 99.4%.

<u>Example 37 (Freebasing):</u>

124 g of Camphersulfonic acid salt was dissolved in toluene (428 g), MeTHF, (48 g) and water (425 g) together with 2 g of sodium hydrogen carbonate. 49.3 g of 20% w/w aquous sodium hydroxide solution were added dropwise to increase the pH to 10. The mixture was heated to 40-50 °C, filtered clear, and the phases were separated. The organic layer was washed at 40-50 °C with a 5% w/w aqueous solution of sodium hydrogen carbonate (210 mL) and then evaporated to dryness to yield 70.5 g of **Ia'-1** as a beige solid (99% yield, purity: 98%w/w.; 99.4% ee (Chiralpak IC column, heptane/ethanol gradient (with 0.02% of diethylamine as stabilizing additive), 1 mL/min, 220nm). Altematively, the distillation can be stopped before completion to obtain **Ia'-1** as a 50%w/w solution in toluene.

<u>Example 38</u>

The reaction was performed under inert gas atmosphere. All solvents and solutions for the reaction and work-up procedure were degassed with nitrogen prior to use. Compound (**II'-1**) (93.9% purity, 78.5 g, 251 mmol) and catalyst **(Va-3)** (0.817 g, 1.25 mmol, 0.5 mol%) were placed in a round bottom flask under inert gas atmosphere (nitrogen). Acetonitrile (302 mL) was added and the mixture was mixed for 3 h under a constant flow of nitrogen to get a brownish to red solution **(Solution 1).** Sodium formate (85.4 g, 1256 mmol) was dissolved in degassed water (537 mL). The solution was further degassed by bubbling through nitrogen **(Solution 2). Solution 2** was placed in a reactor followed by **Solution 1.**

The mixture was heated up to 35 °C within about 35 minutes and stirred for 6 h. Process control showed full conversion of the starting material **(II'-1).** The reaction mixture was cooled down to 25 °C and transferred into a separation vessel and the phases were separated. From the organic layer, most of the acetonitrile is removed under reduced pressure (150-100 mbar) and a jacket temperature of 40 °C. The aqueous layer was re-extracted with xylene (162 g). The xylene layer is added to the distillation sump of the acetonitrile layer. Again, vacuum is applied (100-40 mbar, 50 °C jacket temperature), removing residues of acetonitrile, water, and part of the xylene (distillate amount: 139 g). At 50 °C, 1 g of seed crystals are added. Then a solution of (1S)-(+)-10-camphor sulfonic acid (58.3 g, 251 mmol) in MeTHF (241 g) is added continuously within 30 minutes at 50 °C under fast stirring. The mixture is kept at that temperature for 30 min, cooled to 10 °C within 2 h and then kept at 10 °C overnight. The mixture is filtered and washed twice with 100 g of MTBE each. The filter cake is dried under vacuum at 30 °C yielding 122 g of Camphersulfonic acid salt (89% yield over two steps) in 97.1% w/w assay purity and with an ee of 99.4%.

## Claims

1. A process for preparing a compound of the formula **(Ia)** or **(Ib),**

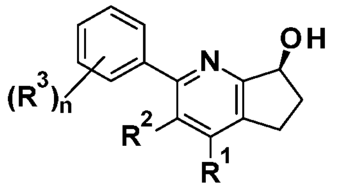

**(Ia)**

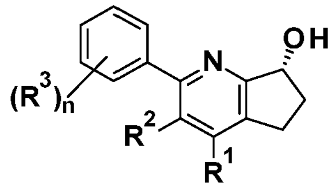

**(Ib)**

where

$R^1$ and $R^2$ are independently from one another selected from the group consisting of hydrogen and $C_1$-$C_4$-alkyl, each $R^3$, if present, is independently selected from $C_1$-$C_4$-alkyl, and n is 0, 1, 2 or 3,

comprising asymmetric transfer hydrogenation of a ketone of the formula (**II**)

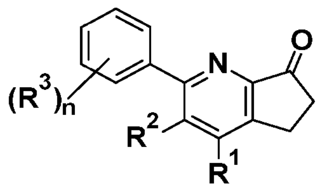

**(II)**

in which the substituents $R^1$, $R^2$, $R^3$ and the integer n are as defined for the compound of the formula **(Ia)** or **(Ib),** in presence of a chiral ruthenium catalyst and a polar solvent, wherein the ruthenium catalyst has the general formula **(Va), (Vb), (VIa)** or **(VIb):**

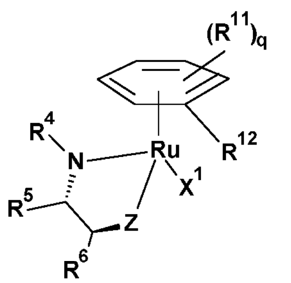

**(Va)**

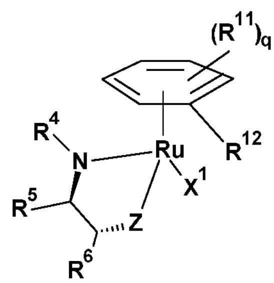

**(Vb)**

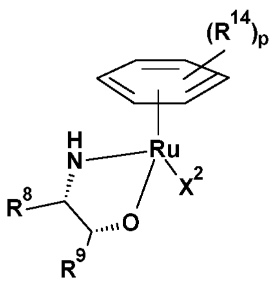

**(VIa)**

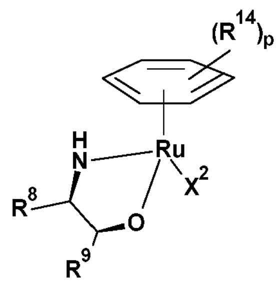

**(VIb)**

where

Z is $NR^{13}$ or O,

$R^4$ is phenylsulphonyl, wherein the phenyl is unsubstituted or substituted with one or more substituents independently selected from $C_1$-$C_4$-alkyl and halogen, or
$R^4$ is 2-pyrrolidinylcarbonyl,
$R^5$ and $R^6$ together form a $-(CH_2)_4-$ group, or
$R^5$ and $R^6$ are unsubstituted phenyl, each $R^{11}$, if present, is independently selected from $C_1$-$C_4$-alkyl,
$R^{12}$ is $C_1$-$C_4$-alkyl or hydrogen and
$R^{13}$ is hydrogen, or
$R^{12}$ and $R^{13}$ form together a $-(CH_2)_3-$, $-(CH_2)_4-$, $-CH_2-O-CH_2-$, $*-(CH_2)_2-O-CH_2-\#$ or $*-(CH_2)-O-(CH_2)_2-\#$ group, where the bond marked with "*" is bonded to the nitrogen and the bond marked with "#" is bonded to the phenyl ring,
q is 0, 1, 2, 3, 4 or 5,
$X^1$ is chlorine or bromine, or
$X^1$ is $BF_4^-$, $PF_6^-$ or $SbF_6^-$, in which case the Ru-$X^1$ bond is of a coordinative or ionic nature and the Ru has a positive charge,
$R^8$ and $R^9$ are unsubstituted phenyl, or
$R^8$ and $R^9$ form together a group of the formula

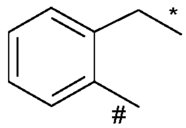

,

wherein the bond identified by "*" is connected to the carbon bearing the hydroxyl group and the bond identified by "#" is connected to the carbon bearing the amino group, each $R^{14}$, if present, is independently selected from $C_1$-$C_4$-alkyl,
p is 0, 1, 2, 3, 4, 5 or 6, and
$X^2$ is chlorine or bromine, or
$X^2$ is $BF_4^-$, $PF_6^-$ or $SbF_6^-$, in which case the Ru-$X^2$ bond is of a coordinative or ionic nature and the Ru has a positive charge.

2. The process according to claim 1, wherein the compounds of formulae **(Ia), (Ib)** and **(II)** are compounds of formulae **(Ia'), (Ib')** and **(II')**

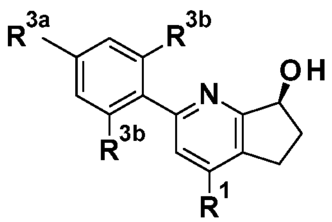

(Ia')

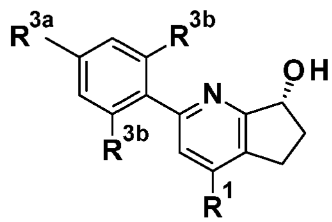

(Ib')

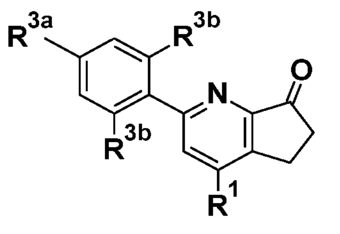

(II'),

wherein $R^1$, $R^{3a}$ and $R^{3b}$ are independently of one another selected from $C_1$-$C_4$-alkyl.

3. The process according to claim 2, wherein

$R^1$ is methyl,
$R^{3a}$ is methyl, and
$R^{3b}$ is ethyl.

4. The process according to any of the preceding claims, wherein the hydrogen source is selected from the group consisting of sodium formate, potassium formate, lithium formate, calcium formate, magnesium formate, formic acid/triethylamine, potassium tert-butylate/isopropanol, sodium tert-butylate/isopropanol and lithium tert-butylate/isopropanol.

5. The process according to any of the preceding claims, wherein the hydrogen source is sodium formate or formic acid/triethylamine.

6. The process according to any of the preceding claims, wherein the amount of ruthenium catalyst used is within the range of from 0.1 mol% to 5 mol%, based on the amount of the compound of the formula **(II).**

7. The process according to any of the preceding claims, wherein the transfer hydrogenation is conducted at a temperature within the range of from 20°C to 80°C.

8. The process according to any of the preceding claims, wherein the polar solvent is selected from the group consisting of dichloromethane, methanol, ethanol, isopropanol, *n*-butanol, tetrahydrofuran, 2-methyl-tetrahydrofuran, dimethylformamide, acetonitrile, methanol/water, ethanol/water, isopropanol/water, *n*-butanol/water, tetrahydrofuran/water, 2-methyl-tetrahydrofuran/water, dimethylformamide/water, acetonitrile/ water, and mixtures thereof.

9. The process according to claim 1, wherein $R^{12}$ is $C_1$-$C_4$-alkyl and Z is O or NH, and wherein the chiral ruthenium catalyst is formed *in situ* by mixing a dichloro (aromatic ligand)ruthenium (II) dimer precatalyst or a dibromo (aromatic ligand)ruthenium (II) dimer precatalyst with a chiral ligand of the formula **(IIIa'), (IIIb'), (IVa)** or **(IVb),**

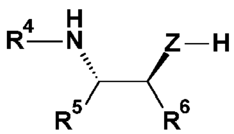

**(IIIa')**

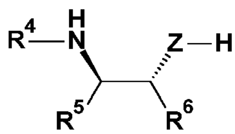

**(IIIb')**

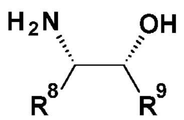

**(IVa)**

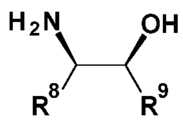

**(IVb),**

wherein

$R^4$, $R^5$ and $R^6$ are each as defined for the complexes of the formulae **(Va)** and **(Vb),**
Z is NH or O,
$R^8$ and $R^9$ are each as defined for the complexes of the formulae **(VIa)** and **(VIb),**
and wherein the aromatic ligand of the precatalyst is selected from the group consisting of p-cymene and benzene, which is optionally substituted with one or more methyl groups,
in an organic solvent.

10. The process according to any of the preceding claims, wherein the product of formula **(Ia)** or **(Ib)** or a mixture thereof is purified by forming a crystalline addition salt with camphor sulfonic acid.

11. The process according to any of the preceding claims, wherein the ketone of formula (**II**) is obtained from a racemic mixture of the compounds of the formulae **(Ia)** and **(Ib)**

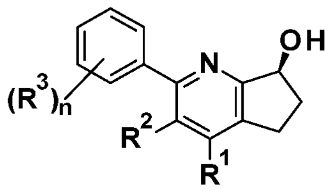

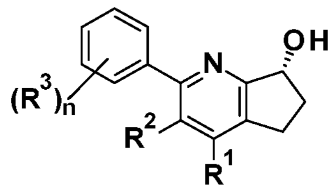

(Ia) (Ib),

wherein the substituents $R^1$, $R^2$, $R^3$ and the integer n are each as defined for the compound of the formula (**II**), by oxidation using TEMPO, a TEMPO derivative or a TEMPO analogue, a hypochlorite salt and optionally a bromide salt.

**Patentansprüche**

**1.** Verfahren zur Herstellung einer Verbindung der Formel (Ia) oder (Ib),

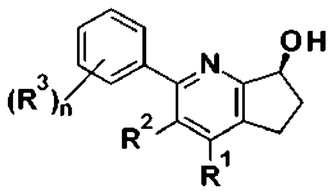

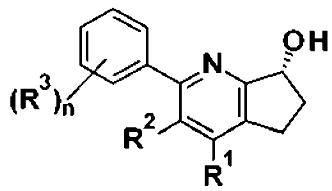

(Ia) (Ib)

wobei

$R^1$ und $R^2$ unabhängig voneinander aus der Gruppe bestehend aus Wasserstoff und $C_1$-$C_4$-Alkyl ausgewählt sind,
$R^3$, falls vorhanden, jeweils unabhängig aus $C_1$-$C_4$-Alkyl ausgewählt ist und
n für 0, 1, 2 oder 3 steht,
umfassend die asymmetrische Transferhydrierung eines Ketons der Formel (II)

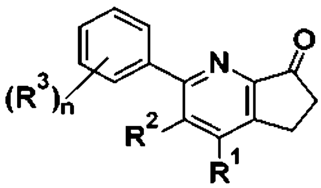

(II)

in der die Substituenten $R^1$, $R^2$, $R^3$ und die ganze Zahl n wie für die Verbindung der Formel (Ia) oder (Ib) definiert sind,
in Gegenwart eines chiralen Rutheniumkatalysators und eines polaren Lösungsmittels, wobei der Rutheniumkatalysator die allgemeine Formel (Va), (Vb), (VIa) oder (VIb) aufweist:

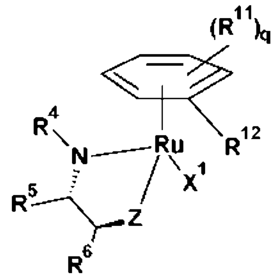

(Va)

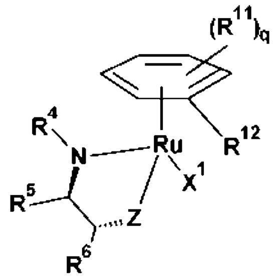

(Vb)

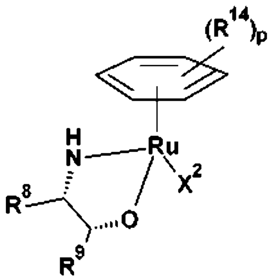

(VIa)

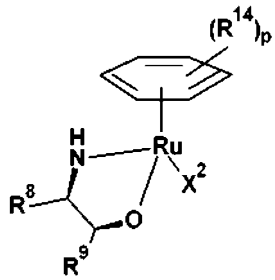

(VIb)

wobei
Z für $NR^{13}$ oder O steht,
$R^4$ für Phenylsulfonyl steht, wobei das Phenyl unsubstituiert oder durch einen oder mehrere Substituenten, die unabhängig aus $C_1$-$C_4$-Alkyl und Halogen ausgewählt sind, substituiert ist, oder
$R^4$ für 2-Pyrrolidinylcarbonyl steht,
$R^5$ und $R^6$ zusammen eine $-(CH_2)_4-$ Gruppe bilden oder
$R^5$ und $R^6$ für unsubstituiertes Phenyl stehen, $R^{11}$, falls vorhanden, jeweils unabhängig aus $C_1$-$C_4$-Alkyl ausgewählt ist,
$R^{12}$ für $C_1$-$C_4$-Alkyl oder Wasserstoff steht und
$R^{13}$ für Wasserstoff steht oder
$R^{12}$ und $R^{13}$ zusammen eine - $(CH_2)_3$-, - $(CH_2)_4$-, $-CH_2-O-CH_2-$, *- $(CH_2)_2-O-CH_2$-#- oder *-$(CH_2)-O-(CH_2)_2$-#-Gruppe bilden, wobei die mit "*" markierte Bindung an den Stickstoff gebunden ist und die mit "#" markierte Bindung an den Phenylring gebunden ist,
q für 0, 1, 2, 3, 4 oder 5 steht,
$X^1$ für Chlor oder Brom steht oder
$X^1$ für $BF_4^-$, $PF_6^-$ oder $SbF_6^-$ steht, wobei die Ru-$X^1$-Bindung in diesem Fall koordinativ oder ionisch ist und das Ru eine positive Ladung aufweist,
$R^8$ und $R^9$ für unsubstituiertes Phenyl stehen oder
$R^8$ und $R^9$ zusammen eine Gruppe der Formel

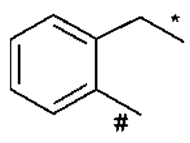

bilden, wobei die durch "*" identifizierte Bindung mit dem die Hydroxylgruppe tragenden Kohlenstoff verbunden ist und die durch "#" identifizierte Bindung mit dem die Aminogruppe tragenden Kohlenstoff verbunden ist,
$R^{14}$, falls vorhanden, jeweils unabhängig aus $C_1$-$C_4$-Alkyl ausgewählt ist,
p für 0, 1, 2, 3, 4, 5 oder 6 steht und
$X^2$ für Chlor oder Brom steht oder
$X^2$ für $BF_4^-$, $PF_6^-$ oder $SbF_6^-$ steht, wobei die Ru-$X^2$-Bindung in diesem Fall koordinativ oder ionisch ist und das Ru eine positive Ladung aufweist.

**2.** Verfahren nach Anspruch 1, wobei es sich bei den Verbindungen der Formeln (Ia), (Ib) und (II) um Verbindungen der Formeln (Ia'), (Ib') und (II') handelt:

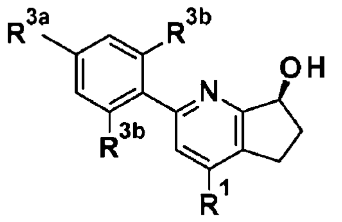

(Ia')

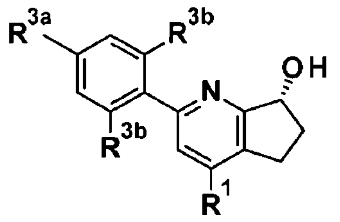

(Ib')

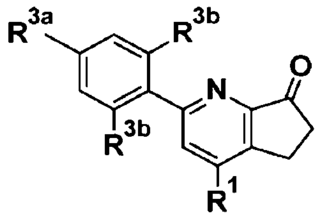

(II'),

wobei $R^1$, $R^{3a}$ und $R^{3b}$ unabhängig voneinander aus $C_1$-$C_4$-Alkyl ausgewählt sind.

**3.** Verfahren nach Anspruch 2, wobei

$R^1$ für Methyl steht,
$R^{3a}$ für Methyl steht und
$R^{3b}$ für Ethyl steht.

**4.** Verfahren nach einem der vorhergehenden Ansprüche, wobei die Wasserstoffquelle aus der Gruppe bestehend aus Natriumformiat, Kaliumformiat, Lithiumformiat, Calciumformiat, Magnesiumformiat, Ameisensäure/Triethylamin, Kalium-tert.-butylat/Isopropanol, Natrium-tert.-butylat/Isopropanol und Lithium-tert.-butylat/Isopropanol ausgewählt wird.

**5.** Verfahren nach einem der vorhergehenden Ansprüche, wobei es sich bei der Wasserstoffquelle um Natriumformiat oder Ameisensäure/Triethylamin handelt.

**6.** Verfahren nach einem der vorhergehenden Ansprüche, wobei die Menge an eingesetztem Rutheniumkatalysator im Bereich von 0,1 Mol-% bis 5 Mol-%, bezogen auf die Menge der Verbindung der Formel (II), liegt.

**7.** Verfahren nach einem der vorhergehenden Ansprüche, wobei die Transferhydrierung bei einer Temperatur im Bereich von 20 °C bis 80 °C durchgeführt wird.

**8.** Verfahren nach einem der vorhergehenden Ansprüche, wobei das polare Lösungsmittel aus der Gruppe bestehend aus Dichlormethan, Methanol, Ethanol, Isopropanol, n-Butanol, Tetrahydrofuran, 2-Methyltetrahydrofuran, Dimethylformamid, Acetonitril, Methanol/Wasser, Ethanol/Wasser, Isopropanol/Wasser, n-Butanol/Wasser, Tetrahydrofuran/Wasser, 2-Methyltetrahydrofuran/Wasser, Dimethylformamid/Wasser, Acetonitril/Wasser und Mischungen davon ausgewählt wird.

**9.** Verfahren nach Anspruch 1, wobei $R^{12}$ für $C_1$-$C_4$-Alkyl steht und Z für O oder NH steht und wobei der chirale Rutheniumkatalysator in situ durch Mischen eines Dichloro(aromatischer Ligand)ruthenium(II)-dimer-Präkatalysators oder eines Dibromo(aromatischer Ligand)ruthenium(II)-dimer-Präkatalysators mit einem chiralen Liganden der Formel (IIIa'), (IIIb'), (IVa) oder (IVb),

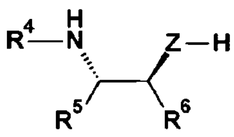 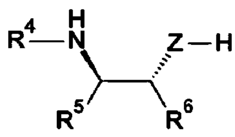

(IIIa') (IIIb')

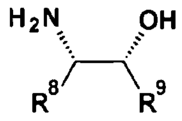 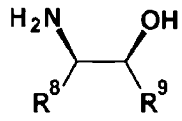

(IVa) (IVb),

wobei

$R^4$, $R^5$ und $R^6$ jeweils wie für die Komplexe der Formeln (Va) und (Vb) definiert sind,
Z für NH oder O steht,
$R^8$ und $R^9$ jeweils wie für die Komplexe der Formeln (VIa) und (VIb) definiert sind
und wobei der aromatische Ligand des Präkatalysators aus der Gruppe bestehend aus p-Cymol und Benzol, das gegebenenfalls durch eine oder mehrere Methylgruppen substituiert ist, ausgewählt wird,
in einem organischen Lösungsmittel gebildet wird.

**10.** Verfahren nach einem der vorhergehenden Ansprüche, wobei das Produkt der Formel (Ia) oder (Ib) oder ein Gemisch davon durch Bilden eines kristallinen Additionssalzes mit Camphersulfonsäure gereinigt wird.

**11.** Verfahren nach einem der vorhergehenden Ansprüche, wobei das Keton der Formel (II) aus einem racemischen Gemisch der Verbindungen der Formeln (Ia) und (Ib)

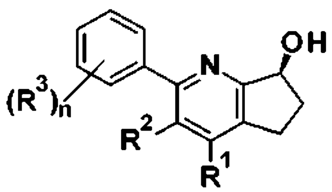 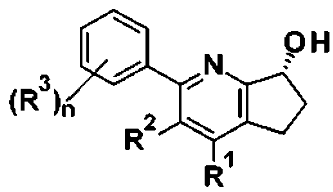

(Ia) (Ib),

wobei die Substituenten $R^1$, $R^2$, $R^3$ und die ganze Zahl n jeweils wie für die Verbindung der Formel (II) definiert sind,
durch Oxidation mit TEMPO, einem TEMPO-Derivat oder einem TEMPO-Analogon, einem Hypochloritsalz und gegebenenfalls einem Bromidsalz erhalten wird.

## Revendications

**1.** Procédé de préparation d'un composé de la formule (Ia) ou (Ib),

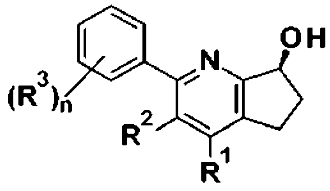

(Ia)

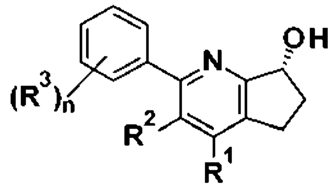

(Ib)

où

$R^1$ et $R^2$ sont indépendamment l'un de l'autre choisis dans un groupe constitué d'hydrogène et de $C_1$-$C_4$-alkyle,
chaque $R^3$, s'il est présent, est indépendamment choisi parmi un $C_1$-$C_4$-alkyle, et
n est 0, 1, 2 ou 3,
comprenant une hydrogénation asymétrique par transfert d'une cétone de la formule (II)

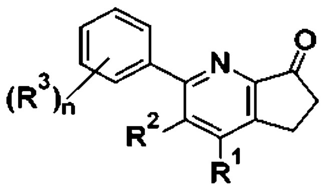

(II)

dans laquelle les substituants $R^1$, $R^2$, $R^3$ et l'entier n sont tels que définis pour le composé de la formule (Ia) ou (Ib), en présence d'un catalyseur chiral au ruthénium et d'un solvant polaire, dans lequel le catalyseur au ruthénium a la formule générale (Va), (Vb), (VIa) ou (VIb) :

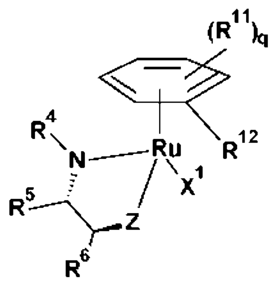

(Va)

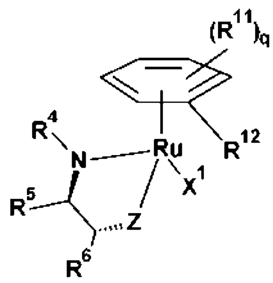

(Vb)

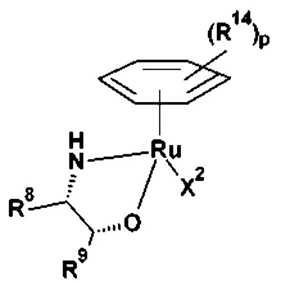

(VIa)

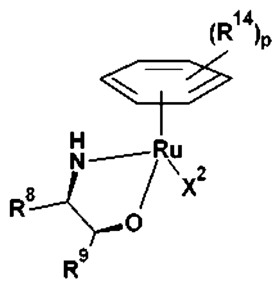

(VIb)

où
Z est $NR^{13}$ ou 0,
$R^4$ est phénylsulfonyle, dans lequel le phényle est non substitué ou substitué par un ou plusieurs substituants indépendamment choisis parmi le $C_1$-$C_4$-alkyle et l'halogène, ou
$R^4$ est 2-pyrrolidinylcarbonyle,

$R^5$ et $R^6$ forment conjointement un groupe $-(CH_2)_4-$, ou

$R^5$ et $R^6$ sont phényle non substitué, chaque $R^{11}$, s'il est présent, est indépendamment choisi parmi un $C_1$-$C_4$-alkyle, $R^{12}$ est $C_1$-$C_4$-alkyle ou hydrogène et

$R^{13}$ est hydrogène, ou

$R^{12}$ et $R^{13}$ forment conjointement un groupe $-(CH_2)_3-$, - $(CH_2)_4-$, $-CH_2-O-CH_2-$, $*-(CH_2)_2-O-CH_2-\#$ ou $*-(CH_2)-O-(CH_2)_2-\#$, où la liaison marquée par « * » est liée à l'azote et la liaison marquée par « # » est liée au cycle phényle, q est 0, 1, 2, 3, 4 ou 5,

$X^1$ est chlore ou brome, ou

$X^1$ est $BF_4^-$, $PF_6^-$ ou $SbF_6^-$, auquel cas la liaison Ru-$X^1$ est d'une nature coordinante ou ionique et le Ru a une charge positive,

$R^8$ et $R^9$ sont phényle non substitué, ou

$R^8$ et $R^9$ forment conjointement un groupe de la formule

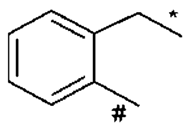,

dans laquelle la liaison identifiée par « * » est reliée au carbone portant le groupe hydroxyle et la liaison identifiée par « # » est reliée au carbone portant le groupe amino,

chaque $R^{14}$, s'il est présent, est indépendamment choisi parmi un $C_1$-$C_4$-alkyle,

p est 0, 1, 2, 3, 4, 5 ou 6, et

$X^2$ est chlore ou brome, ou

$X^2$ est $BF_4^-$, $PF_6^-$ ou $SbF_6^-$, auquel cas la liaison Ru-$X^2$ est de nature coordinante ou ionique et le Ru a une charge positive.

**2.** Procédé selon la revendication 1, dans lequel les composés des formules (Ia), (Ib) et (II) sont des composés des formules (Ia'), (Ib') et (II')

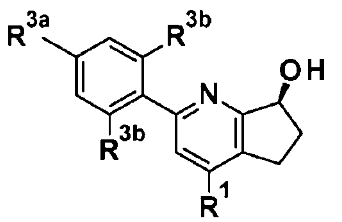

**(Ia')**

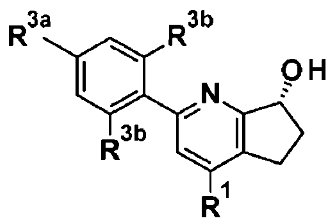

**(Ib')**

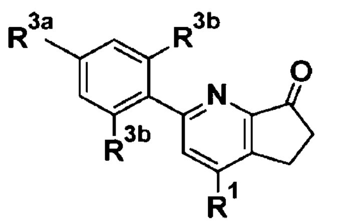

**(II')**,

dans lesquelles $R^1$, $R^{3a}$ et $R^{3b}$ sont indépendamment l'un de l'autre choisis parmi un $C_1$-$C_4$-alkyle.

**3.** Procédé selon la revendication 2, dans lequel

$R^1$ est méthyle,

$R^{3a}$ est méthyle, et

$R^{3b}$ est éthyle.

**4.** Procédé selon l'une quelconque des revendications précédentes, dans lequel la source d'hydrogène est choisie dans un groupe constitué du formiate de sodium, du formiate de potassium, du formiate de lithium, du formiate de calcium, du formiate de magnésium, de l'acide formique/triéthylamine, du tert-butylate de potassium/isopropanol, du tert-butylate de sodium/isopropanol et du tert-butylate de lithium/isopropanol.

**5.** Procédé selon l'une quelconque des revendications précédentes, dans lequel la source d'hydrogène est le formiate de sodium ou l'acide formique/triéthylamine.

**6.** Procédé selon l'une quelconque des revendications précédentes, dans lequel la quantité de catalyseur au ruthénium utilisée est comprise entre 0,1 % en moles et 5 % en moles, par rapport à la quantité du composé de la formule (II).

**7.** Procédé selon l'une quelconque des revendications précédentes, dans lequel l'hydrogénation par transfert est conduite à une température comprise entre 20 °C et 80 °C.

**8.** Procédé selon l'une quelconque des revendications précédentes, dans lequel le solvant polaire est choisi dans un groupe constitué du dichlorométhane, du méthanol, de l'éthanol, de l'isopropanol, du n-butanol, du tétrahydrofurane, du 2-méthyl-tétrahydrofurane, du diméthylformamide, de l'acétonitrile, du méthanol/eau, de l'éthanol/eau, de l'isopropanol/eau, du n-butanol/eau, du tétrahydrofurane/eau, du 2-méthyl-tétrahydrofurane/eau, du diméthylformamide/eau, de l'acétonitrile/eau, et de leurs mélanges.

**9.** Procédé selon la revendication 1, dans lequel $R^{12}$ est un $C_1$-$C_4$-alkyle et Z est O ou NH, et dans lequel le catalyseur chiral au ruthénium est formé *in situ* par mélange d'un précatalyseur dimère de dichloro(ligand aromatique) ruthénium (II) ou d'un précatalyseur dimère de dibromo(ligand aromatique)ruthénium (II) avec un ligand chiral des formules (IIIa'), (IIIb'), (IVa) ou (IVb),

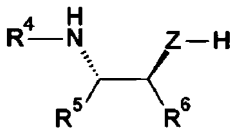

(IIIa')

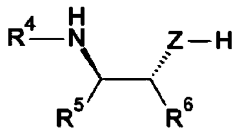

(IIIb')

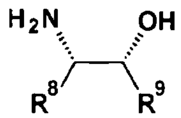

(IVa)

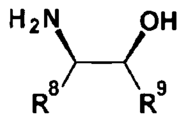

(IVb),

dans lesquelles

$R^4$, $R^5$ et $R^6$ sont chacun tels que définis pour les complexes des formules (Va) et (Vb),
Z est NH ou O,
$R^8$ et $R^9$ sont chacun tels que définis pour les complexes des formules (VIa) et (VIb),
et dans lequel le ligand aromatique du précatalyseur est choisi dans un groupe constitué du p-cymène et du benzène, qui est éventuellement substitué par un ou plusieurs groupes méthyle,
dans un solvant organique.

**10.** Procédé selon l'une quelconque des revendications précédentes, dans lequel le produit de la formule (Ia) ou (Ib) ou un mélange de ceux-ci est purifié par formation d'un sel d'addition cristallin avec l'acide camphre sulfonique.

**11.** Procédé selon l'une quelconque des revendications précédentes, dans lequel la cétone de la formule (II) est obtenue à partir d'un mélange racémique des composés des formules (Ia) et (Ib)

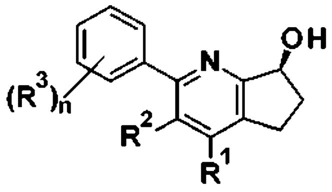

(Ia)

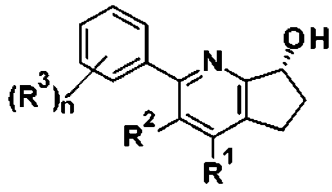

(Ib),

dans lequel les substituants $R^1$, $R^2$, $R^3$ et l'entier n sont chacun tels que définis pour le composé de la formule (II), par oxydation en utilisant du TEMPO, un dérivé de TEMPO ou un analogue de TEMPO, un sel d'hypochlorite et éventuellement un sel de bromure.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019185541 A
- EP 0654464 A
- WO 2011162397 A
- WO 2012084812 A
- WO 2015197530 A


### Non-patent literature cited in the description


- **S. KAISER et al.** *Angew. Chem. Int. Ed.*, 2006, vol. 45, 5194-5197
- **D. H. WOODMANSEE et al.** *Chem. Sci.*, 2010, vol. 1, 72-78
- **C. MAZET et al.** *Org. Lett.*, 2006, vol. 8, 1879-1882
- **A. NAIK et al.** *Chem. Commun.*, 2010, vol. 46, 4475-4477
- *Tetrahedron: Asymmetry*, 2009, vol. 20, 1425-1432
- **R. HODGKINSON et al.** *Organometallics*, 2014, vol. 33, 5517-5524
- **V. PAREKH et al.** *Catal. Sci. & Technol.*, 2012, vol. 2, 406-414
- **M. SHIBUYA ; M TOMIZAWA ; I. SUZUKI ; Y. IWABUCHI**. *J. Am. Chem. Soc.*, 2006, vol. 128, 8412-8413